# EUROPEAN PATENT APPLICATION

(11) **EP 4 666 997 A1**
(43) Date of publication of application: **24.12.2025**
(21) Application number: 24752631.2
(22) Date of filing: 04.01.2024
(51) Int. Cl.: A61K 8/9728, A61Q 19/00, C12N 1/06, C12R 1/84, C12R 1/865

(54) **PRODUCTION PROCESS FOR PRODUCING YEAST CELL WALL EXTRACT HYDROLYSATE USING ORGANIC ACIDS**

(30) Priority: 09.02.2023 CN 202310088199
(71) Applicant: Beijing Active Blue Crystal Biotechnology Co., Ltd., Beijing 102204 (CN)
(72) Inventor: LING, Feng, Beijing 102204 (CN); DING, Wenyong, Beijing 102204 (CN); SONG, Yanxia, Beijing 102204 (CN)
(74) Representative: Mathys & Squire
(86) International application number: PCT/CN2024/070486
(87) International publication number: WO 2024/164773

(57) **Abstract**

The present invention discloses a process for producing yeast cell wall extract hydrolysate using organic acid. The process removes color and odor substances and inorganic salts carried by yeast after fermentation and cultivation through preliminary hydrolysis with acetic acid and purifies the yeast cell wall extract at the same time. Then, the yeast cell wall extract from which color and odor substances and inorganic salts have been removed is hydrolyzed with formic acid to make the produced yeast cell wall extract hydrolysate more easily soluble in water. Through the above organic acid hydrolysis process, the present invention provides a production process for a colorless, odorless, salt-free and easily soluble product containing substances in yeast cell walls that are beneficial to skin protection, repair and treatment. The present invention also provides a method for removing and recycling the organic acids added in the process, thereby reducing production costs and reducing pollution.

## Description

### FIELD OF THE INVENTION

The present invention belongs to the field of biotechnology, and more specifically relates to a process for producing yeast cell wall extract hydrolysate using organic acid.

### BACKGROUND OF THE INVENTION

The yeast cell wall accounts for about 20%-30% of the total cell dry weight and plays an important role in maintaining cell morphology and intercellular recognition. According to the structure, the yeast cell wall can be divided into 4 layers, the inner layer is the glucan layer, the middle layer is mainly composed of protein, the outer layer is the mannan layer, and the innermost layer is chitin. The layers can be partially inlaid; according to the chemical composition, mannan accounts for about 30% of the yeast cell wall dry weight, β -glucan accounts for about 30%, glycoprotein and chitin account for about 20%, and other components such as protein, lipids, inorganic salts, etc. account for about 20%.

β -glucan is a structural polysaccharide, connected to the plasma membrane, and constitutes the main component of the yeast cell wall. Its function is to support the external mannan. β -glucan is composed of β -1,3-glucan and β -1,6-glucan, and the ratio of the two is 85:15. β -Glucan has β -1,3-glucan as the skeleton, β -1,6-glucan as the branch chain, and the innermost layer is chitin.

The average molecular weight of β -1,3-glucan is 240KDa, the average molecular weight of β -1,6-glucan is 24KDa, the average molecular weight of mannan is 150KDa, and the average molecular weight of chitin is 25KDa.

Mannan has multiple physiological functions such as cell recognition and control of cell wall pore size. Mannan is covalently linked to protein. The main chain is a single chain, and multiple mannoses are connected by α-1,6-glycosidic bonds. The mannose side chains are connected to the main chain by α-1,2 and α-1,3 bonds, and some side chains are combined with functional groups related to yeast cell antigens.

The two confirmed functions of mannan in yeast cell wall are adsorption of pathogens and regulation of immunity.

Mannan is widely used in cosmetics. It increases the viscosity of aqueous solutions, stabilizes emulsions, has moisturizing effects, and has skin-revitalizing and antiinflammatory properties.

The mannoprotein in yeast cell wall has the same hydrophilic and lipophilic structure as surfactants and many emulsifiersand can act as an emulsifier in cosmetics.

Yeast glucan is an immune polysaccharide existing in the cell wall of natural yeast. It was first discovered in 1963 that it has anti-tumor activity. Later, it was found that it has outstanding performance in antibacterial, immune regulation, anti-radiation, gastrointestinal regulation, helping tissue structure regeneration or repair, promoting wound healing, preventing cardiovascular and cerebrovascular diseases and diabetes. It has unique biological activities in tumors, hepatitis, cardiovascular, diabetes, lowering blood lipids, anti-aging, etc. Yeast glucan is the first glucan found to have immune activity. Yeast glucan is widely used in cosmetics. It mainly has functions such as film formation, adhesive, emollient, and skin conditioning.

The basic unit of chitin is acetylglucosamine, which is a polymer composed of 1000 to 3000 acetylglucosamine residues connected by 1,4-glycoside chains.

Chitin can be hydrolyzed into acetyl glucosamine, which acts as a moisturizer and skin conditioner in cosmetics. It can also accelerate wound healing, improve skin hydration, reduce fine lines, improve skin roughness, resist the synthesis of tyrosinase, reduce the production of melanin, improve uneven skin tone, and have a certain whitening effect.

The yeast cell wall also contains proteins, lipids and other substances that are beneficial to the growth of skin cells. They play a beneficial role in skin and growth metabolism.

As mentioned above, yeast cell walls contain a large amount of beneficial substances that can be used in cosmetics. However, yeast produces yellow or brownish yellow substances during the fermentation and cultivation process and also produces a special smell. This color and smell will seriously affect the quality of cosmetics when used as cosmetic raw materials. At the same time, yeast cell walls also contain inorganic salts and other substances that are harmful to the skin. Therefore, in order to produce yeast cell wall extracts suitable for application in the field of cosmetics, it is necessary to remove color and odor substances and inorganic salts.

In addition, as mentioned above, yeast polysaccharides include β-glucan and α -mannan. The average molecular weight of β-1,3-glucan is 240KDa, the average molecular weight of β-1,6-glucan is 24Kda, the average molecular weight of mannan is 150KDa, and the average molecular weight of chitin is 25Kda. These macromolecular polysaccharides are difficult to dissolve in water. When used as cosmetic raw materials, their solubility is a very important indicator, so improving their solubility is very important for their application in the field of cosmetics.

In the existing methods for producing yeast polysaccharides, the main methods are the combination of alkali and enzyme, the combination of alkali and salt, the combination of alkali and mineral acids, the combination of alkali and organic solvent, the combination of mineral acids and salts, etc. Usually, the products produced by these methods are single yeast cell wall polysaccharides, such as β-glucan and α-mannan. These methods destroy and lose alarge number of substances in the yeast cell wall that are beneficial to skin care, reducing the function of the produced products in cosmetic applications. Due to the single product, the utilization rate of the yeast cell wall raw materials is greatly reduced, and the yield of the product is also reduced. These destroyed and lost substances will also cause environmental pollution. Due to the single products produced by these methods, the production cost of the products is also increased.

More importantly, none of these methods provides a method for recycling the chemicals used, and the alkalis, salts and enzymes used in these methods are very difficult to recycle, or even impossible to recycle. If these chemicals are not recycled, they will damage the environment. Moreover, these alkalis, salts and enzymes constitute the main cost of production, and their loss will greatly increase the production cost.

Therefore, it is very important to find a production process for a colorless, odorless, salt-free and easily soluble product containing substances in yeast cell walls that are beneficial to skin protection, repair and treatment, and to recycle the chemicals used, thereby forming an environmentally friendly and low-cost production process.

### BRIEF DESCRIPTION OF THE DRAWINGS

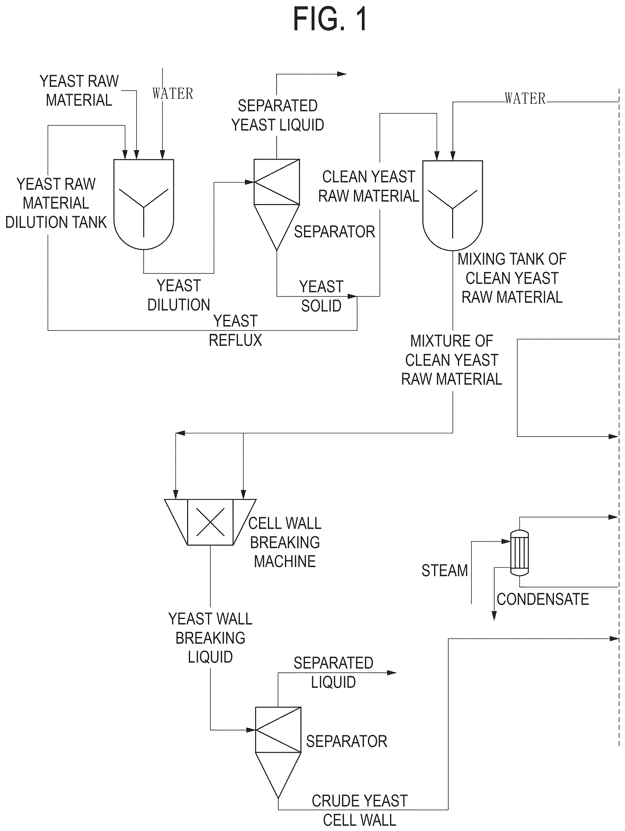

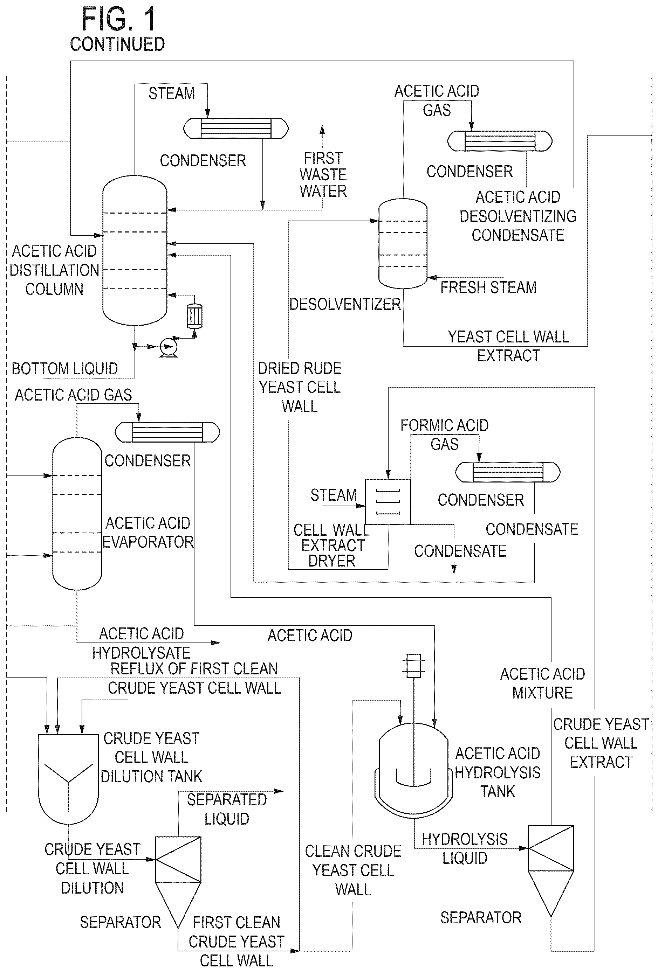

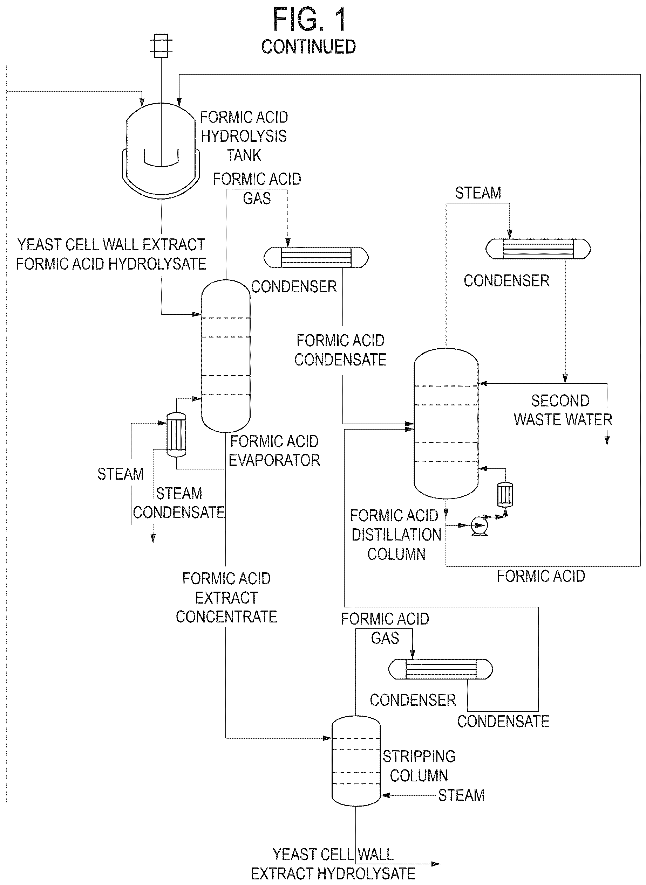

Fig. 1 is a detailed flowchart of the process herein disclosed.

### DETAILED DESCRIPTION

The objective of the present disclosure is to find a process for producing a colorless, odorless, salt-free and easily soluble product containing substances in yeast cell walls that are beneficial to skin protection, repair and treatment, and at the same time, the chemicals used can be recycled, thereby forming an environmentally friendly and low-cost production process.

In several embodiments, the objective of the present dislcosure is achieved by the following technical scheme:
A process for producing yeast cell wall extract hydrolysate using organic acid, comprising the following steps:
(1) adding water to yeast raw material, preferably mixing evenly, to obtain a yeast dilution, followed by separation of the obtained yeast dilution using a separator to separate and removing the liquid part to obtain yeast solid; and
(2) repeating step (1) at least once to obtain clean yeast raw material; and
(3) breaking the yeast cell wall of the clean yeast raw material to obtain a yeast wall-breaking liquid; and
(4) using a separator to separate the yeast wall-breaking liquid, separating and removing the liquid part, to obtain crude yeast cell wall; and
(5) adding water to the crude yeast cell wall, preferably mixing evenly, to obtain a crude yeast cell wall dilution; and
(6) using a separator to separate the crude yeast cell wall dilution again and removing the liquid part to obtain the first clean crude yeast cell wall; and
(7) repeating steps (4)-(6) at least once, to preliminary remove color and odor substances and other impurities to obtain clean crude yeast cell walls; and
(8) adding the clean crude yeast cell walls and acetic acid to an acetic acid hydrolysis tank to obtain a clean crude yeast cell wall and acetic acid mixture; and
(9) hydrolyzing the clean crude yeast cell wall and acetic acid mixture to further hydrolyze the color and odor substances and other impurities that can be dissolved by acetic acid in the clean crude yeast cell walls to obtain a clean crude yeast cell wall hydrolysate; and
(10) using a separator to separate the clean crude yeast cell wall hydrolysate, and removing the acetic acid liquid, to obtain an acetic acid mixture and a precipitated crude yeast cell wall extract; and
(11) drying the precipitated crude yeast cell wall extract to obtain a dried crude yeast cell wall extract and an acetic acid drying condensate; and
(12) desolventizing the dried crude yeast cell wall extract by the desolventizer to obtain a yeast cell wall extract and an acetic acid desolventizing condensate; and
(13) distilling the acetic acid mixture, the acetic acid drying condensate and the acetic acid desolventizing condensate to obtain a distilled acetic acid liquidform distillation column bottom and a first distillation wastewater; and
(14) evaporating the distilled acetic acid liquid to obtain recycled acetic acid and an acetic acid hydrolysate; and
(15) reusing the recycled acetic acid to the acetic acid hydrolysis tank; and
(16) adding theyeast cell wall extract and formic acid to the formic acid hydrolysis tank to obtain amixture of yeast cell wall extract and formic acid; and
(17) hydrolyzing mixture of yeast cell wall extract and formic acid to obtain a yeast cell wall extract formic acid hydrolysate;
(18) Evaporating the yeast cell wall extract formic acid hydrolysate to obtain a yeast cell wall extract formic acid hydrolysate concentrate and a formic acid evaporation condensate;
(19) Stripping the yeast cell wall extract formic acid hydrolysate concentrate to obtain a yeast cell wall extract hydrolysate and a formic acid stripping condensate;
(20) Distilling the formic acid evaporation condensate and the formic acid stripping condensate to obtain distilled formic acid and a second distillation wastewater;
(21) Reusing the distilled formic acid to the formic acid hydrolysis tank.

In some aspects, the yeast in step (1) comprises Saccharomyces cerevisiae, Pichia pastoris, or the like.

In some aspects, the dry matter content of the yeast raw material in steps (1), (2) and (3) is not less than about 30% (w/w), calculated as the weight percentage of the dry matter contained in the yeast raw material.

In some aspects, the ratio of water to yeast solids in step (1) is not less than about 2 times (w/w).

In some aspects, the repeating step (1) in step (2) is not less than 2 times.

In some aspects, the separator in steps (1), (4) and (6) can separate the solid and liquid phases in the separated liquid, and the separator optionally comprises a horizontal screw centrifuge, a disc separator, a plate and frame filter press, or the like.

In some aspects, the method of breaking the yeast cell wall in step (3) comprises mechanical breaking, chemical breaking and/or enzymatic breaking.

In some aspects, the ratio of water to crude yeast cell wall in step 5 is not less than about 2 times (w/w).

In some aspects, repeating steps (4)-(6) in step (7) is not less than 2 times.

In some aspects, in step (8), the ratio of acetic acid to clean crude yeast cell wall is not less than about 2 times (w/w).

In some aspects, hydrolyzing the clean crude yeast cell wall in step (9), the temperature of hydrolyzing is not less than about 65 ° C.

In some aspects, hydrolyzing the clean crude yeast cell wall in step (9), the time of hydrolyzing is not less than about 1 hour.

In some aspects, the acetic acid liquid in step (10) includes the color and odor substances and other impurities in clean crude yeast cell wall that can be dissolved by acetic acid.

In some aspects, the acetic acid content in the yeast cell wall extract is less than about 0.1%, calculated as the weight percentage of acetic acid to the weight percentage of the yeast cell wall extract.

In some aspects, the acetic acid hydrolysate in step (14) includes the color and odor substances and other impurities in the clean crude yeast cell wall that can be dissolved by acetic acid.

In some aspects, addition of the yeast cell wall extract and formic acid to the formic acid hydrolysis tank in step (16), comprises a ratio of formic acid to yeast cell wall extract of not less than about 1 times (w/w).

In some aspects, during the hydrolyzing mixture of yeast cell wall extract in step 7, the temperature for hydrolyzing is not less than about 70° C.

In some aspects, during the hydrolyzing mixture of yeast cell wall extract in step 7, the time for hydrolyzing is not less than about 0.5 hours.

In some aspects, the formic acid content in the yeast cell wall extract hydrolysate in step (19) is less than about 200 ppm, calculated as the weight percentage of formic acid in the yeast cell wall extract hydrolysate.

In some aspects, the viscosity value (representing solubility) of the yeast cell wall extract hydrolysate in step (19) does not exceed about 500 cP, preferably the viscosity value is detected by a viscosity tester at a detection temperature of about 20° C, and the dry matter content in the yeast cell wall extract hydrolysate is about 35%, calculated as the percentage of the dry matter weight of the yeast cell wall extract hydrolysate in the total weight of the yeast cell wall extract hydrolysate.

In some aspects, the absorbance value (representing chromaticity) of the yeast cell wall extract hydrolysate in step (19) is not greater than about 0.05, preferably the absorbance value is detected by spectrophotometry, with ultrapure water as a control, by detecting the absorbance value of the yeast cell wall extract hydrolysate obtained at a wavelength of 420 nm.

In some aspects, the inorganic salts content of the yeast cell wall extract hydrolysate in step (19) is less than about 500 ppm, and preferably the inorganic salt content is calculated using the ash content in the yeast cell wall extract hydrolysate, which is calculated based on the percentage of the ash weight in the yeast cell wall extract hydrolysate to the total dry matter weight of the yeast cell wall extract hydrolysate.

The advantages and beneficial effects of the present disclosure include:
First, the product (yeast cell wall extract hydrolysate) produced by the present invention retains most of the substances in the yeast cell wall that are beneficial to skin protection. While retaining the products produced by the existing production technology, such as yeast glucan and mannan, other beneficial components in the yeast cell wall, such as acetyl glucosamine, protein and lipids, are added, thereby improving the effects of the produced product in the field of cosmetics.

Second since the product of the present invention retains most of the substances in the yeast cell wall that are beneficial to skin protection, compared with the prior art, the utilization rate of the yeast cell wall raw materials is improved, therefore, the product yield is improved and the production cost is reduced.

Third, the production process adopted by the present invention adopts simple process steps, mainly using acetic acid hydrolysis to remove the yellow or brownish yellow color and odor substances produced by yeast during the cultivation and fermentation process, reducing the production cost and improving the effect of the product produced in the cosmetics field.

Fourth, the production process adopted by the present invention adopts simple process steps, mainly using acetic acid hydrolysis to remove the yellow or brownish yellow color and odor substances produced by yeast during cultivation and fermentation, reducing production costs and improving the effect of the produced products in the field of cosmetics.

Fifth, the chemical substances added in the production process of the present invention are organic acids (formic acid and acetic acid). Compared with the chemical substances (mainly alkali, salt, enzyme) added in the existing process, the boiling point of organic acids is much lower, the boiling point of formic acid is 100.6°C, and the boiling point of acetic acid is 117.9°C. The chemical substances added in the prior art cannot be removed by simple physical methods due to their high boiling points, which results in increased sewage treatment costs and environmental pollution. In steps (10), (11) and (12), the present production process adopts the separation, drying and desolventizing methods used in the traditional chemical industry to remove acetic acid from the intermediate product. In steps (18) and (19), the evaporation and stripping methods used in the traditional chemical industry are adopted to remove formic acid from the intermediate product of yeast cell wall extract formic acid hydrolysate, thereby overcoming the problems existing in the existing process.

Sixth, the chemical substances added in the production process of the present invention are organic acids (formic acid and acetic acid). Compared with the chemical substances (mainly alkali, salt, enzyme) added in the existing process, organic acids are not only easy to remove, but also have the greater advantage of being able to be recycled. In steps (13) and (14) of the present invention, the distillation and evaporation methods used in the traditional chemical industry are adopted to recover acetic acid. In step (15), acetic acid is reused in the production process of the present invention. In step (20), the distillation method used in the traditional chemical industry is adopted to recover formic acid. In step (21), formic acid is reused in the production process of the present invention. The recycling of acetic acid and formic acid not only reduces production costs, but also reduces environmental pollution, thereby overcoming the problems existing in the existing process.

### EXAMPLES

The present invention will be further described below through specific examples.

### EXAMPLE 1

(1) 600g of water was added to 200g of waste yeast (dry matter content is 33%), which is a byproduct of beer fermentation, the separator speed was set to 5000 rpm, with the separation factor of 4472, and separated for 15 minutes. The supernatant was removed to obtain 198g of yeast solids at the bottom (dry matter content was 33%);
(2) 600g of water was added to the yeast solids, stirred evenly, and the separator speed was set to 5000 rpm, with the separation factor of 4472, and separated for 15 minutes. The liquid on the top was removed to obtain 197g of yeast solids (dry matter content is 33%);
(3) Step (2) was repeated 4 times to obtain 194g of clean yeast raw material (dry matter content is 33%);
(4) 300g of water was added to the clean yeast raw material, stirred evenly, and then a homogenizer was used to break the clean yeast raw material, the homogenizer pressure was set to 130MPa, the flow rate was 55ml/min, the number of cycles was 5 times, and the yeast wall breaking liquid was obtained;
(5) The yeast wall breaking liquid was separated, with the separator speed set at 5500 rpm, the separation factor was 5411, and the separation occured for 15 minutes. The liquid part was removed to obtain 79g of separated crude yeast cell wall (dry matter content is 34%);
(6) 250g of water was added to the separated crude yeast cell wall, stirred evenly, and then separated again to remove the liquid, obtaining 78g of crude yeast cell wall (dry matter content is 34%);
(7) Steps (5) and (6) were repeated 3 times. Through this separation and washing, most of the color and odor substances, inorganic salts and other impurities were removed, and 76g of clean crude yeast cell wall (dry matter content is 34%) was obtained;
(8) 250g of acetic acid was added to the clean crude yeast cell wall and initial hydrolysis was performed for 1 hour, the color and odor substances and other impurities that can be dissolved by acetic acid in the clean crude yeast cell wall were further hydrolyzed, and the inorganic salts therein were further released to obtain a clean crude yeast cell wall acetic acid hydrolysate;
(9) The clean crude yeast cell wall acetic acid hydrolysate was separated, with the separator speed set at 5500 rpm, the separation factor was 5411, and the separation time was 15 minutes. The acetic acid liquid was removed to obtain 68g of precipitated crude yeast cell wall extract (dry matter content is 34%) and 258g of acetic acid mixed solution;
(10) The precipitated crude yeast cell wall extract was dried to obtain 27 g of dried crude yeast cell wall extract (dry matter content is 85%) and 41 g of acetic acid dried condensate;
(11) The dried crude yeast cell wall extract was desolventized to obtain 27 g of yeast cell wall extract (dry matter content of 86%) and 21 g of acetic acid desolventized condensate;
(12) The acetic acid mixed solution, acetic acid dried condensate and acetic acid desolventized condensate was distilled to obtain 255 g of acetic acid tower bottom liquid and 64 g of first distillation wastewater;
(13) The acetic acid tower bottom liquid was evaporated to obtain 249 g of acetic acid and 6 g of acetic acid hydrolysate;
(14) 30g of the yeast cell wall extract was added into the formic acid, preliminarily hydrolyzed at 80°C for 1 hour to obtain yeast cell wall extract formic acid hydrolysate;
(15) The yeast cell wall extract formic acid hydrolysate was evaporated to obtain 42g yeast cell wall extract formic acid hydrolysate concentrate (dry matter content of 55%) and 15g formic acid evaporation condensate;
(16) The yeast cell wall extract formic acid hydrolysate concentrate was stripped to obtain 61g formic acid stripping condensate and 39g decolorized, deodorized, desalted and soluble yeast cell wall extract hydrolysate (dry matter content of 60%);
(17) The formic acid evaporation condensate and formic acid stripping condensate was distilled to obtain 29g formic acid and 46g second distillation wastewater.

Detection of yeast cell wall extract hydrolysate:
Color: Spectrophotometry was used, and the equipment was a UV-visible spectrophotometer produced by INESA, modelL5S. Pure water was used as a control, and the absorbance of the yeast cell wall extract hydrolysate at a wavelength of 420nm was detected, and the absorbance value obtained was 0.04.

Visual inspection showed that it was almost colorless.

Smell: Sensory measurement showed that the yeast cell wall extract hydrolysate had almost no special smell of yeast.

Viscosity detection (reflecting the degree of hydrolysis): Viscosity tester detection method was used, and the equipment was a viscosity tester, modelNDJ-5S. When the dry matter content of the yeast cell wall extract hydrolysate was 35%, the detection result at 20° C was 445cP.

Determination of inorganic salts: Using a conductivity meter (Raymag desktop conductivity meter DDS-307A), the test result was 0.078S/m, and the salt content in the yeast cell wall extract hydrolysate was 388ppm.

Determination of acetic acid content in yeast cell wall extract: Using liquid chromatography to detect, the test result was 0.09%, calculated according to the weight percentage of acetic acid in the yeast cell wall extract.

Determination of formic acid content in yeast cell wall extract hydrolysate: Using liquid chromatography to detect, the test result was191ppm, calculated according to the weight percentage of formic acid in the yeast cell wall extract hydrolysate.

### EXAMPLE 2

(1) 800g of water was added to 200g of waste yeast (dry matter content is 33%), which is a byproduct of beer fermentation, and the separator speed was set to 5000 rpm, the separation factor was 4472, and separation occured for 15 minutes, after which the supernatant was removed to obtain 197g of yeast solids at the bottom (dry matter content is 33%);
(2) 800g of water was added to the yeast solids, stirred evenly, the separator speed was set to 5000 rpm, the separation factor was 4472, and separation occured for 15 minutes, after which the liquid on the top was removed to obtain yeast solids. 196g (dry matter content was 33%);
(3) Step (2) was repeated 4 times to obtain 192g of clean yeast raw material (dry matter content is 33%);
(4) 200g of water was added to the clean yeast raw material, stirred evenly, and then a homogenizer was used to break the clean yeast raw material. The homogenizer pressure was set to 130MPa, the flow rate was 55ml/min, and the number of cycles was 5 times to obtain yeast wall breaking liquid;
(5) The yeast wall breaking liquid was separated with the separator speed set at 5000 rpm. The separation factor was 4472, and the separation was carried out for 15 minutes. The liquid part was removed to obtain 81g of crude yeast cell wall (dry matter content is 33%);
(6) 800g of water was added to the separated crude yeast cell wall, stirred evenly, and then separated again to remove the liquid to obtain 80g of crude yeast cell wall (dry matter content was 33%);
(7) Steps (5) and (6) were repeated 4 times. Through this separation and washing, most of the color and odor substances, inorganic salts and other impurities were removed to obtain 77g of clean crude yeast cell wall (dry matter content is 33%).
(8) 800g acetic acid was added to the clean crude yeast cell wall, and preliminary hydrolysis was performed at 90°C for 2 hours, further hydrolyzed the color and odor substances and other impurities that can be dissolved by acetic acid in the clean crude yeast cell wall, and further released the inorganic salts therein to obtain a clean crude yeast cell wall acetic acid hydrolysate;
(9) The clean crude yeast cell wall acetic acid hydrolysate was separated, with the separator speed set at 5500 rpm, the separation factor was 5411, and separation occured for 15 minutes. The acetic acid liquid was removed to obtain 67g of precipitated crude yeast cell wall extract (dry matter content of 34%) and 810g of acetic acid mixed solution;
(10) The precipitated crude yeast cell wall extract was dried to obtain 27g of dried crude yeast cell wall extract (dry matter content of 85%) and 40g of acetic acid dried condensate;
(11) The dried crude yeast cell wall extract was desolventized to obtain 26g of yeast cell wall extract (dry matter content of 87%) and 21g of acetic acid desolventized condensate;
(12) The acetic acid mixed solution, acetic acid dried condensate and acetic acid desolventized condensate was distilled to obtain 811g of acetic acid tower bottom liquid and 60g of first distillation wastewater;
(13) The acetic acid tower bottom liquid was evaporated to obtain 798g of acetic acid and 12g of acetic acid hydrolysate.
(14) 73g formic acid was added to the yeast cell wall extract and preliminary hydrolysis was performed at 90°C for 3 hours to obtain a formic acid hydrolysate of the yeast cell wall extract;
(15) The formic acid hydrolysate of the yeast cell wall extract was evaporated to obtain 42g of a formic acid hydrolysate concentrate of the yeast cell wall extract (with a dry matter content of 55%) and 58g of a formic acid evaporation condensate;
(16) The formic acid hydrolysate concentrate of the yeast cell wall extract was stripped to obtain 60g of a formic acid stripping condensate and 38g of a decolorized, deodorized, desalted and soluble yeast cell wall extract hydrolysate (with a dry matter content of 60%);
(17) The formic acid evaporation condensate and the formic acid stripping condensate were distilled to obtain 72g of formic acid and 45g of second distillation wastewater.

Detection of yeast cell wall extract hydrolysate:
Color: Spectrophotometry was used, and the equipment was a UV-visible spectrophotometer produced by INESA, model L5S. Pure water was used as a control, and the absorbance of the yeast cell wall extract hydrolysate obtained at a wavelength of 420nm was detected. The absorbance value obtained was 0.03.

Visual inspection showed that it was colorless.

Odor: Sensory measurement was used, and the yeast cell wall extract hydrolysate did not have the special smell of yeast.

Viscosity detection (reflecting the degree of hydrolysis): The viscosity tester detection method was used, and the equipment was a viscosity tester, model NDJ-5S. When the dry matter content of the yeast cell wall extract hydrolysate was 35%, the detection result at 20° C was 272cP.

Determination of inorganic salts: Using a conductivity meter (Raymag desktop conductivity meter DDS-307A), the test result was 0.074S/m, and the salt content in the yeast cell wall extract hydrolysate was 368ppm.

Determination of acetic acid content in yeast cell wall extract: Using liquid chromatography to detect, the test result was 0.08%, calculated according to the weight percentage of acetic acid in the yeast cell wall extract.

Determination of formic acid content in yeast cell wall extract hydrolysate: Using liquid chromatography to detect, the test result was 182ppm, calculated according to the weight percentage of formic acid in the yeast cell wall extract hydrolysate.

### EXAMPLE 3

1) 1000g of water was added to 200g of wet yeast (dry matter content is 33%) that has just been cultured, the separator was set to 5000 rpm, the separation factor was 4472, and separation occurred for 15 minutes, after which the supernatant was removed to obtain 196g of yeast solid at the bottom (dry matter content is 33%);
(2) 1000g of water was added to the yeast solid, stir evenly, separate speed was set at 5000 rpm, the separation factor was 4472, and separation occured for 15 minutes, after which the liquid on the top was removed to obtain 195g of yeast solid (dry matter content is 33%);
(3) Step (2) was repeated 5 times to obtain 191g of clean yeast raw material (dry matter content is 33%);
(4) 250g of water was added to the clean yeast raw material, stirred evenly, and then a homogenizer was used to break the clean yeast raw material. The homogenizer pressure was set to 130MPa, the flow rate was 55ml/min, and the number of cycles was 5 times to obtain yeast wall breaking liquid;
(5) The yeast wall breaking liquid was separeated with the separator speed set at 5000 rpm. The separation factor was 4472, and the separation was carried out for 15 minutes. The liquid part was removed to obtain 80g of crude yeast cell wall (dry matter content is 33%);
(6) 1000g of water was added to the separated crude yeast cell wall, stirred evenly, and then separated again to remove the liquid to obtain 79g of crude yeast cell wall (dry matter content is 33%);
(7) Steps (5) and (6) were repeated 5 times. Through this separation and washing, most of the color and odor substances, inorganic salts and other impurities were removed to obtain 75g of clean crude yeast cell wall (dry matter content is 33%of the total mass content);
(8) 1000g of acetic acid was added to the clean crude yeast cell wall and preliminary hydrolysis was performed at 100° C for 3 hours to further hydrolyze the color and odor substances and other impurities that can be dissolved by acetic acid in the clean crude yeast cell wall, and further release the inorganic salts therein to obtain a clean crude yeast cell wall acetic acid hydrolysate;
(9) The clean crude yeast cell wall acetic acid hydrolysate was separated, with the separator speed set at 5500 rpm, the separation factor was 5411, and separation occured for 15 minutes. The acetic acid liquid was removed to obtain 66g of precipitated crude yeast cell wall extract (dry matter content of 34%) and 1009g of acetic acid mixed solution;
(10) The precipitated crude yeast cell wall extract was dried to obtain 26g of dried crude yeast cell wall extract (dry matter content of 85%) and 39g of acetic acid dried condensate;
(11) The dried crude yeast cell wall extract was desolventized to obtain 26g of yeast cell wall extract (dry matter content of 86%) and 20g of acetic acid desolventized condensate;
(12) The acetic acid mixed solution, acetic acid drying condensate and acetic acid desolventized condensate were distilled to obtain 1013g of acetic acid tower bottom liquid and 56g of first distillation wastewater;
(13) The acetic acid tower bottom liquid was evaporated to obtain 998g of acetic acid and 15g of acetic acid hydrolysate;
(14) 213g of formic acid was added to the yeast cell wall extract and preliminary hydrolysis was performed at 100° C for 5 hours to obtain formic acid hydrolysate of yeast cell wall extract;
(15) The formic acid hydrolysate was evaporated to obtain 41g of yeast cell wall extract formic acid hydrolysate concentrate (dry matter content of 55%) and 198g of formic acid evaporation condensate;
(16) The yeast cell wall extract formic acid hydrolysate concentrate was stripped to obtain 58g of formic acid stripping condensate and 37g of decolorized, deodorized, desalted and soluble yeast cell wall extract hydrolysate (dry matter content of 60%);
(17) The formic acid evaporation condensate and the formic acid stripping condensate were distilled to obtain 212g of formic acid and 45g of second distillation wastewater.

Detection of yeast cell wall extract hydrolysate:
Color: Spectrophotometry was used, and the equipment was a UV-visible spectrophotometer produced by INESA, model numberL5S. Pure water was used as a control, and the absorbance value of the yeast cell wall extract hydrolysate obtained at a wavelength of 420nm was detected. The absorbance value obtained was 0.02.

Visual inspection showed that it was colorless.

Odor: Sensory measurement was used, and the yeast cell wall extract hydrolysate did not have the special smell of yeast.

Viscosity detection: The viscosity tester detection method was used, and the equipment was a viscosity tester, model number NDJ-5S. When the dry matter content of the yeast cell wall extract hydrolysate was 35%, the detection result at 20° C was 130cP.

Determination of inorganic salts: Using a conductivity meter (Raymag desktop conductivity meter DDS-307A), the test result was 0.066S/m, and the salt content in the yeast cell wall extract hydrolysate was 328ppm.

Determination of acetic acid content in yeast cell wall extract: Using liquid chromatography, the test result was 0.06%, calculated according to the weight percentage of acetic acid in the yeast cell wall extract.

Determination of formic acid content in yeast cell wall extract hydrolysate: Using liquid chromatography, the test result was 171ppm, calculated according to the weight percentage of formic acid in the yeast cell wall extract hydrolysate.

## Claims

1. A process for producing yeast cell wall extract hydrolysate using organic acid, comprising the following steps of:
(1) adding water to a yeast raw material to obtain a yeast dilution, followed by separation of the obtained yeast dilution using a separator and removing the liquid fraction to obtain a yeast solid; and
(2) repeating step (1) at leat once to obtain clean yeast raw material; and
(3) breaking the yeast cell wall of the clean yeast raw material to obtain a yeast wall-breaking liquid; and
(4) using a separator to separate the yeast wall-breaking liquid, separating and removing the liquid part, to obtain a crude yeast cell wall; and
(5) adding water to the crude yeast cell wall to obtain a crude yeast cell wall dilution;
and
(6) separating the crude yeast cell wall dilution using a separator and removing the liquid fraction to obtain a first clean crude yeast cell wall; and
(7) repeating steps (4)-(6), to preliminarily remove color and odor substances and other impurities to obtain clean crude yeast cell walls; and
(8) adding the clean crude yeast cell walls and acetic acid to an acetic acid hydrolysis tank to obtain a clean crude yeast cell wall and acetic acid mixture; and
(9) hydrolyzing the clean crude yeast cell wall and acetic acid mixture to further hydrolyze the color and odor substances and other impurities that can be dissolved by acetic acid in the clean crude yeast cell walls to obtain a clean crude yeast cell wall hydrolysate; and
(10) using a separator to separate the clean crude yeast cell wall hydrolysate to obtain an acetic acid mixture and a precipitated crude yeast cell wall extract; and
(11) drying the precipitated crude yeast cell wall extract to obtain a dried crude yeast cell wall extract and an acetic acid drying condensate; and
(12) desolventizing the dried crude yeast cell wall extract by a desolventizer to obtain a yeast cell wall extract and an acetic acid desolventizing condensate; and
(13) distilling the acetic acid mixture, the acetic acid drying condensate and the acetic acid desolventizing condensate to obtain a distilled acetic acid liquid from distillation column bottom and a first distillation wastewater; and
(14) evaporating the distilled acetic acid liquid to obtain recycled acetic acid and an acetic acid hydrolysate; and
(15) reusing the recycled acetic acid to the acetic acid hydrolysis tank; and
(16) adding the yeast cell wall extract and formic acid to the formic acid hydrolysis tank to obtain a mixture of yeast cell wall extract and formic acid. and
(17) hydrolyzing mixture of yeast cell wall extract and formic acid to obtain a yeast cell wall extract formic acid hydrolysate; and
(18) Evaporating the yeast cell wall extract formic acid hydrolysate to obtain a yeast cell wall extract formic acid hydrolysate concentrate and a formic acid evaporation condensate; and
(19) Stripping the yeast cell wall extract formic acid hydrolysate concentrate to obtain a yeast cell wall extract hydrolysate and a formic acid stripping condensate; and
(20) Distilling the formic acid evaporation condensate and the formic acid stripping condensate to obtain distilled formic acid and a second distillation wastewater; and
(21) Reusing the distilled formic acid to the formic acid hydrolysis tank.

2. The process according to claim 1, wherein the yeast in step (1) is selected from Saccharomyces cerevisiae and Pichia pastoris.

3. The process according to claim 1, wherein the dry matter content of the yeast raw material in steps (1), (2) and (3) is not less than about 30% (w/w), calculated as the weight percentage of the dry matter contained in the yeast raw material.

4. The process according to claim 1, wherein the separator in steps (1), (4) and (6) can separate the solid and liquid phases in the separated liquid, and is selected from a horizontal screw centrifuge, a disc separator, a plate and frame filter press.

5. The process according to claim 1, wherein the method of the breaking the yeast cell wall in step (3) is selected from mechanical breaking, chemical breaking and enzymatic breaking.

6. The process according to claim 1, wherein the acetic acid liquid in step (10) includes the color and odor substances and other impurities in clean crude yeast cell wall that can be dissolved by acetic acid.

7. The process according to claim 1, wherein the acetic acid content in the yeast cell wall extract in step (12) is less than about 0.1%, calculated as the weight percentage of acetic acid to the weight percentage of the yeast cell wall extract.

8. The process according to claim **1,** wherein the acetic acid hydrolysate in step (14) includes the color and odor substances and other impurities in the clean crude yeast cell wall that can be dissolved by acetic acid.

9. The process according to claim 1, wherein the formic acid content in yeast cell wall extract hydrolysate in step (19) is less than about 200 ppm, calculated as the weight percentage of formic acid in the yeast cell wall extract hydrolysate.

10. The process according to claim 1, wherein the viscosity value of the yeast cell wall extract hydrolysate in step (19) does not exceed about 500 cP, wherein the viscosity value is detected by a viscosity tester at a detection temperature of 20° C, and the dry matter content in the yeast cell wall extract hydrolysate is about 35%, calculated as the percentage of the dry matter weight of the yeast cell wall extract hydrolysate in the total weight of the yeast cell wall extract hydrolysate, wherein the viscosity value represents solubility.

11. The process according to claim **1,** wherein the absorbance value (representing chromaticity) of the yeast cell wall extract hydrolysate in step (19) is not greater than about 0.05, wherein the absorbance value is detected by spectrophotometry, with ultrapure water as a control, by detecting the absorbance value of the yeast cell wall extract hydrolysate obtained at a wavelength of 420 nm.

12. The process according to claim 1, wherein the inorganic salts content of the yeast cell wall extract hydrolysate in step (19) is less than about 500 ppm, and wherein the inorganic salt content is calculated using the ash content in the yeast cell wall extract hydrolysate, which is calculated based on the percentage of the ash weight in the yeast cell wall extract hydrolysate to the total dry matter weight of the yeast cell wall extract hydrolysate.
